# EUROPEAN PATENT SPECIFICATION

(11) **EP 1 512 374 B1**
(45) Date of publication and mention of the grant of the patent: **21.06.2006**
(21) Application number: 03077765.0
(22) Date of filing: 03.09.2003
(51) Int. Cl.: A61B 5/18, B60R 16/02

(54) **Method and device for estimating workload for a driver of a vehicle**
Verfahren und Gerät zur Bestimmung der Arbeitsbelastung eines Fahrzeugfahrers
Procédé et dispositif pour l'estimation de la charge de travail d'un conducteur dans un véhicule

(43) Date of publication of application: 09.03.2005
(73) Proprietor: Ford Global Technologies, LLC., Dearborn MI 48126 (US)
(72) Inventor: Engström, Johan #c/o Malmquist, 41657 Göteborg (SE)
(74) Representative: Ekström, Nils

(56) References cited:
- DE-A- 19 723 922
- US-A1- 2002 120 371
- US-A1- 2003 050 740

## Description

### TECHNICAL FIELD

The invention relates to a method for estimating workload for a driver of a vehicle according to the preamble of claim 1. The invention further relates a workload estimator according to the preamble of claim 8.

### BACKGROUND ART

Workload is defined as the portion of a driver's limited capacity which is required to handle a specific traffic scenario. Workload depends on situation demand and also on driver characteristics. The driver characteristics include slowly changing variables such as age and driving skills, temporary driver state such as fatigue, alcohol influence and emotional state and further invariable characteristics such as talent for driving. Workload is thus a subjective measure of the load on a specific driver. Situation demand, that is the objective load on the driver, includes primary task demand, that is demand related to driving and secondary task demand, which is tasks that are not related to driving. Examples of primary task demand can be gear change, acceleration, braking, steering wheel control etcetera, that is all tasks that are related to driving for handling different traffic situations such as negotiation of intersections and turns, overtaking, avoiding obstacles etc. Examples of secondary task demand can be handling of cellular phones, handling of infotainment systems, handling of climate control, handling of vehicle seat position etcetera.

Workload estimators in the context of the description can be used to both estimate workload and demand.

Workload estimation is normally used in workload management systems where a workload estimator provides information about whether the driver is considered to be under high workload or not. In the event the estimator concludes that the driver is under high workload an information system temporary blocks or reschedules information to be presented to the driver if temporary blocking or rescheduling is allowed for the type of information to be presented. Normally such temporary blocking and rescheduling can be allowed unless the information relates to safety related messages.

Workload estimation is a well established technology. For a more general presentation refer to the reports of the EC projects Generic Intelligent Driving Support; Michon 1993 and Centralised management of Vocal Interfaces; 1999.

Normally workload is estimated on the basis of performance related measures, i.e. the demand of an actual traffic situation. The performance related measures can be estimated from information relating to driving related tasks and non-driving related tasks.

For examples of demand based workload estimation systems references are given to Bellet, T. et al. 2002. "Real-time" Analysis of the Driving Situation in Order to Manage On-board Information. *e-Safety Conference Proceedings,* Lyon.

Secondary task demand, that is distraction related tasks, can be estimated by recording in vehicle system operation or by means of eye- and head movement tracking, see for instance "Integrated Safety System", Delphi 2001, exhibited at Frankfurt motor show 2001.

A workload management system using estimation of demand from driving related tasks and distraction related tasks is given in SE 515699. In the event the workload estimator detects a demanding event a high workload output signal is generated. The high workload signal is received by an information filter, which in is set to block out information messages for a predetermined period of time, regardless of what happens during this period of time. Since the period is predetermined and set from the start of an event generating a high workload condition for the driver, the information filter does not adapt well to real life traffic situations where the high workload remains under different time spans for different situations.

### DISCLOSURE OF INVENTION

Short time scale demanding scenarios may be described in terms of a hierarchy of events, situations and driving environments. These terms are defined as follow:
1. Event
   A short time scale change in demand. This may be related to changes in the vehicle dynamic state (e.g. accelerations or turns) or in vehicle operations requiring attentional demand.
2. Situation
   A cluster of events. For example, manoeuvring through an intersection may consist of a deceleration, a short stop and a turn. A parking situation may consist of several turns and reversals, and operating a radio generally involves several consecutive button presses and/or knob turns.
3. Environment
   A cluster of situations. For example, inner city driving may involve sequences of intersections, roundabouts and parking situations.

At each level, the periods between the events, situations or environments are referred to as gaps.

It is an object of the invention to provide an improved workload estimator, which is able to maintain a high workload output signal under a traffic situation constituted by a cluster of events. It is furthermore an object of the invention to provide a workload estimator which allows high workload to remain under different time spans for different situations These objects are achieved by a method for estimating workload according to the characterising portion of claim 1. The objects are furthermore achieved by a workload estimator according to the characterising portion of claim 8.

The invention makes use of a workload delay time frame, which extends a period in time from end point in time of a demanding event until a workload delay end point in time. A high workload output signal generated under the demanding event is maintained under the workload delay time frame. The workload delay time frame will make sure that a high workload output signal will remain under a situation including a cluster of demanding events each generating a high workload output signal.

By use of the method for estimating workload according to the invention and a workload estimator according to the invention together with a workload delay of adequate length, that is 0.1 - 20 s, preferably 2 - 15 s and yet more preferred 3 - 7 s, the workload estimate will more precisely correspond to the actual workload experienced by a driver.

### BRIEF DESCRIPTION OF DRAWINGS

The invention will be described in further detail with references to drawings, where:
- fig. 1: shows a workload manager using a workload estimator according to the invention,
- fig. 2: shows an illustration of conditional rules for workload computation including a workload delay period,
- fig. 3: shows a CAN bus giving access to signals indicative of tasks requiring driver attention,
- fig. 4: shows an example of a hierarchical taxonomy for describing demanding driving scenarios,
- fig. 5: shows a flow chart for a method for estimating work load according to the invention,
- fig. 6: shows an embodiment of a workload estimator according to the invention,
- fig.7: shows a flow chart for a method for estimating work load according to an embodiment of the invention,
- fig.8: shows a flow chart for a method for handling an incoming call or a sms message according to an embodiment of the invention, and
- fig. 9: shows a vehicle on board arrangement for handling an incoming call or a sms message according to an embodiment of the invention.

### DETAILED DESCRIPTION OF THE INVENTION

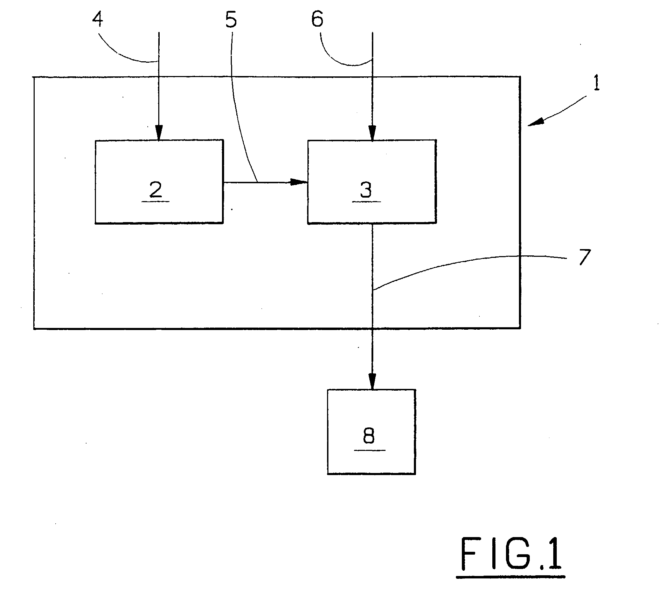

In figure 1 a schematic drawing of a workload manager 1 is shown. The workload manager includes a workload estimator 2 and an information filter 3. The workload estimator 2 receives information regarding tasks requiring driver attention. The information is provided as an input signal 4. The input signal can be of different types including physiological indicators such as heart rate sensors or eye movement indicators, see for instance "Assessment of cardiovascular reactivity by means of spectral analysis", Mulder, L. J. M., PhD thesis, Rijksuniveristetet Groningen, Groningen 1988 or "Automatic Driver Visual Behaviour Measurement", Victor, T., Blomberg, O. And Zelinsky, Vision in vehicles 9, Elsevier Science B.V. Other alternatives for signals that may be used are road map information and information from external sensors such as radar etc.

Preferably the input signal is based on performance related measures which are estimated from information relating to primary task demand and secondary task demand.

The following input signals 4 may be used as a basis for estimation if a demanding event is present:
1. Acceleration
   The vehicle acceleration is obtainable from the vehicle speed signal which is available on a vehicle multiplex system, such as a CAN bus. The signal can also be retrieved from an acceleration sensor.
2. Steering angle
   The steering angle is recorded by a steering angle sensor provided in the steering system. The signal is available on the multiplex system, such as the CAN bus.
3. Steering angle velocity
   The steering angle velocity can be calculated from the steering angle. Preferably the steering angle velocity is obtained from differentiating a filtered steering angle signal.
4. Lateral acceleration
   the lateral acceleration is recorded by a sensor. The signal is available on the multiplex system, such as the CAN bus.
5. Brake pressure
   It is possible to use either only the binary signal of existence of brake pressure or a signal corresponding to the magnitude of the brake pressure. The signal is available on the multiplex system, such as the CAN bus.
6. Turn indicator
   Information regarding use of the turn indicator is available on the multiplex system, such as the CAN bus.
7. Gear position
   Information regarding gear position and or change of gear position is available on the multiplex system, such as the CAN bus.
8. Vehicle navigation system activity buttons.
   Information regarding use of control buttons of the Vehicle navigation system is available on the multiplex system, such as the CAN bus.
9. Infotainment buttons
   Information regarding use of infotainment control buttons, such as phone buttons or audio control buttons is available on the multiplex system, such as the CAN bus.
10. Mirror control buttons
   Information regarding use of mirror control buttons is available on the multiplex system, such as the CAN bus.
11. Window control buttons
   Information regarding use of window control buttons is available on the multiplex system, such as the CAN bus.
12. Seat control buttons
   Information regarding use of seat control buttons is available on the multiplex system, such as the CAN bus.

Further input signals may be used, for instance control buttons for infotainment systems of the vehicle.

In different embodiments of the invention, the person skilled in the art may contemplate to use any combination of the signals available above. The invention is particularly useful for situations where the signal has a varying magnitude and where the magnitude of the signal is compared to a threshold in order to judge if a demanding event is present or not. In these circumstances the length of the period during which the driver is under a high workload condition will vary in time. The workload will remain high during this period and still remain high during a workload delay time frame. Signals 1 - 4 listed above are of this type.

The input signals are used to determine if a demanding event is present or not. A demanding event is an event which takes the driver's attention and where additional requests on the driver's attention, such as informational messages or incoming phone calls (for instance) will be experienced as distracting to the drivers most-important purpose, driving the vehicle.

The following conditions for classifying the signals as indicating a demanding event can be used:

| PARAMETER (TASK) | CONDITION | VALUE |
|---|---|---|
| Positive Longitudinal Acceleration | S> T | 1.5<T<3 m/s² |
| Negative Longitudinal Acceleration | S> T | -2.5<T<-1 m/s² |
| Steering angle threshold | S> T | 90<T<130 degrees |
| Steering angle velocity threshold | S> T | 120<T<170 degrees/s |
| Lateral acceleration | S> T | 2<T<3 m/s² |
| Brake pedal activity | | Brake pedal used |
| Gear lever position | | Gear lever in reverse |
| Turn indicator | | Turn indicator used |
| Vehicle navigation system buttons | | Vehicle navigation system buttons used |
| Phone buttons | | Phone buttons used and/or phone in use |
| Mirror control buttons | | Mirror control buttons used |
| Window control buttons | | Window control buttons used |
| Seat control buttons | | Seat control buttons used |
| Infotainment system buttons | | Infotainment system buttons used |

Here S is signal value and T is the chosen threshold value, which should be chosen within the suggested interval.

If the criteria for a demanding event is fulfilled, the workload estimator generates a high workload output signal 5 which is used as an input signal to the information filter 3.

The workload estimator will continue to generate a high workload output signal under the demanding event and under a workload delay time frame which extends a period in time from the end point in time of the demanding event until a workload delay end point in time.

System tests has shown that a workload delay time frame extending for up to 3 seconds maintains a high workload indication during events, a workload delay time frame extending between 3 and 15 seconds maintains a high workload during situations and a workload delay time frame extending more than 20 second has the possibility of maintaining high workload during demanding driving environments.

In one embodiment of the invention the workload delay time frame is set between 0.1 and 20 seconds, preferably between 2 and 15 seconds and still more preferred between 3 and 7 seconds.

When the workload estimator generates a high workload output signal, the information reschedules or delays system initiated information 6 so that information 7 appears in a controlled fashion via means for communication 8 included in the vehicle.

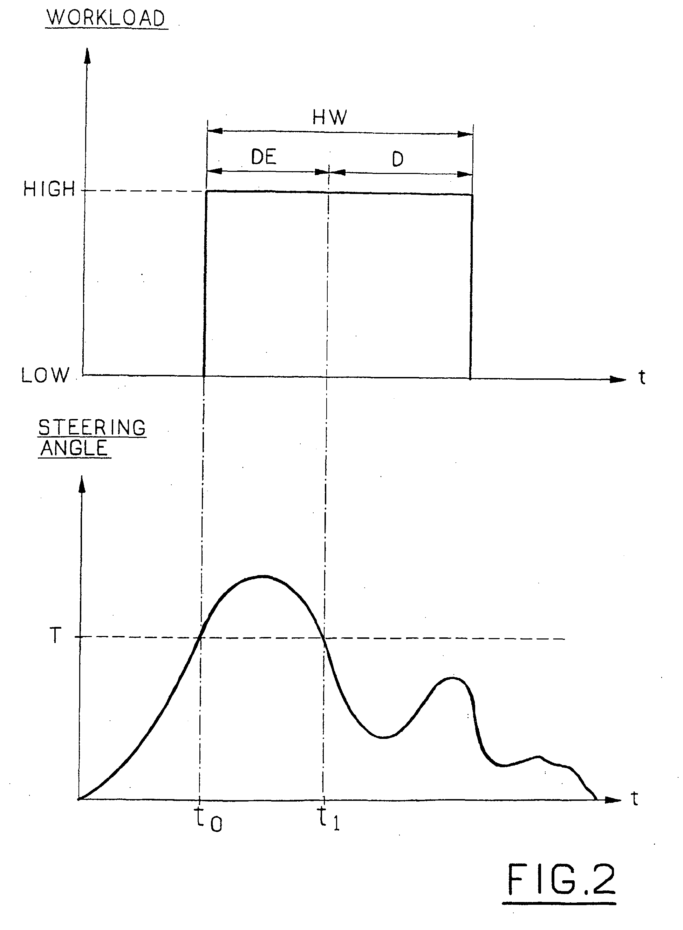

In figure 2 an illustration of conditional rules for workload computation including a workload delay period is shown.

The lower diagram shows the steering angle as a function of the time. A threshold value T indicates the limit over which a demanding event is considered to be present. The demanding event occurs between a demanding event starting point t₀ and a demanding event end point t₁ in time. The workload estimator generates a high workload output signal at the demanding event starting point t₀. In the upper figure it is shown that the high workload output signal HW is maintained under the demanding event DE and under the workload delay time frame D.

The workload delay time frame can be carried out into effect by a counter, which triggers when the demanding event condition is set to fault. The workload delay time frame can have the same duration for all conditions or alternatively have different duration for different conditions. In one embodiment at least two conditions have different duration of the workload delay time frame.

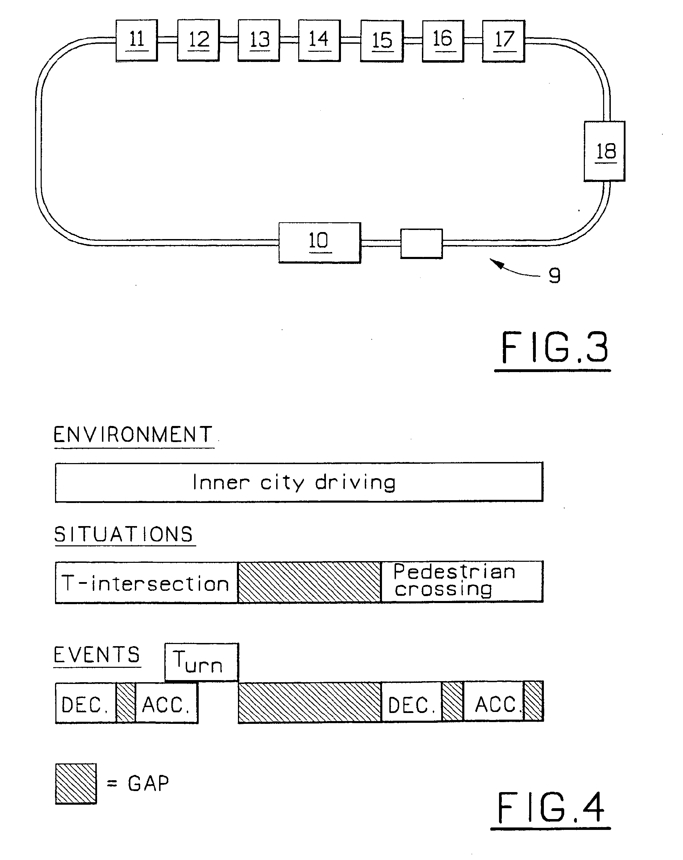

In figure 3 a schematic drawing of a CAN bus 9 in a vehicle is shown. For a general description of CAN bus systems for vehicles see the following U.S. Patents, U.S. Pat. Nos. 5,574,848, 5,551,053, 5,323,385, 5,539,778, 5,600,782, 5,675,830, and 5,448,180. The CAN bus is controlled by a central processor unit 10 which may include the workload estimator 2 and the information filter 3. The CAN bus 9 communicates with different control system in the vehicle such as an engine control unit 11, which provides information about acceleration of the car, a steering control system 12, which provides information about steering angle, a brake control system 13, which provides information about brake pressure, a gear box control system 14, which provides information about gear position, a turn indicator communication switch 15, which provides information about use of the turn indicator, a phone control system 16 , which provides information about the use of the phone, an infotainment control system 17, which provides information about use of control buttons of the infotainment system, furthermore any additional control system 18 may be attached to the bus such as seat control switches, mirror control switches, window control switches, vehicle navigation system control system etc.

By coupling the workload manager 1 to the CAN bus the workload manager can have access to all information of importance for correctly determining if a high workload condition is present or not.

In figure 4 an example of a hierarchical taxonomy for describing demanding driving scenarios is shown.

The lower portion of the figure shows series of events, from left to right a period of deceleration followed by a gap and a period of acceleration together with a sharp turn. An intermediate period, which does not constitute a demanding event, is followed by a deceleration, a gap and a period of an acceleration.

The events are grouped into two different situations: a turn at a T intersection followed by a gap and a pedestrian crossing.

The traffic environment is classified as inner city driving.

The workload delay time frame according to the invention is preferably set to extend so that events making up a situation are grouped together and that a high workload delay will remain under a situation. The workload delay time frame shall preferably not be so long so that separate situations are grouped together. For this reason the workload delay time frame shall have duration of between 2 and 15 seconds, preferably between 3 and 7 seconds.

In a table below a set of different events that should be identified by the workload estimator as demanding events:

| EVENT | DEMAND TYPE | Relevant CAN bus information |
|---|---|---|
| Lane change | Primary task | Turn activity signal |
| Braking when approaching front obstacle | Primary task | Acceleration, brake pedal position, brake pressure |
| Sharp acceleration (For instance at traffic lights or when overtaking.) | Primary task | Acceleration |
| Low speed turn | Primary task | Steering wheel angle, yaw rate, turn indicator signal. |
| High speed curve taking | Primary task | Lateral acceleration |
| Reverse | Primary task | Gear mode |
| Splash on windshield | Primary task | Activity of front wipers |
| Infotainment operation | Secondary task | Infotainment buttons and knobs activity |
| Vehicle navigation system operation | Secondary task | Vehicle navigation system buttons activity |
| Seat adjustment operation | Secondary task | Seat control operation |
| Mirror adjustment operation | Secondary task | Driver mirror control |
| Window adjustment operation | Secondary task | Driver window control |

The demanding events listed above are frequently clustered into demanding situations. Examples of demanding situations are listed below:

| Demanding Situation | Cluster of events |
|---|---|
| Overtaking | Left lane change, acceleration, right lane change |
| Stop and go situations (traffic lights, crosswalks etc) | Deceleration, acceleration |
| intersection | Deceleration, low speed turn, acceleration |
| Roundabout | Deceleration, low speed turn |
| Parking | Reverse, low speed turn |
| Infotainment task execution | Infotainment button presses |
| Vehicle navigation system task execution | Vehicle navigation system button presses |
| Seat adjustment task operation | Seat control operations |
| Mirror adjustment task operation | Mirror control operations |
| Window adjustment task operation | Window control operations |

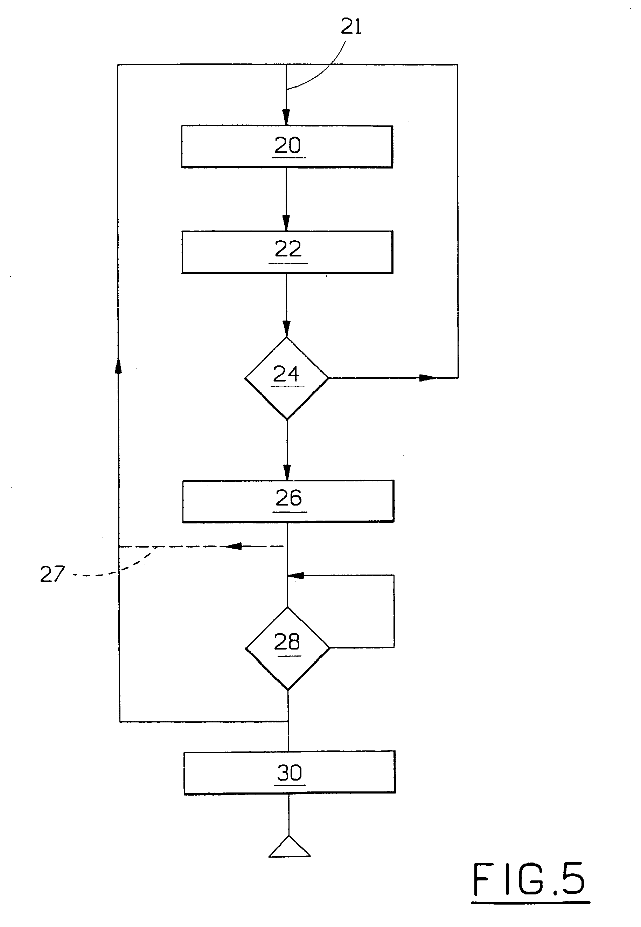

In figure 5 a flow chart for operating a method for estimating workload for a driver of a vehicle is shown.

In a first method step 20 signals 21 indicative of a tasks requiring driver attention are collected. In a second method step 22 the signals are compared with threshold values and/or indication of presence of demanding activity. In a third method step 24 it is determined if a demanding event is present or not. If a demanding event is not present a new set of input signal data is collected. If a demanding event is present a high workload output signal is generated at a fourth method step 26. After the demanding event has been identified it is determined in a fifth method step 28 if the demanding event is still ongoing or if it has ended. The fifth method step is repeated while the demanding event is ongoing. The fifth method step can be replaced with an immediate recollection of a new set of input signal input data, as is indicated by the dashed line. When the demanding event is no longer present the workload delay time frame is started at a sixth method step 30. Different time frames can be used for different tasks. New data are collected under the running of the workload delay time frame. A new identified demanding event will reset the workload delay time frame, which will again start to run after the new demanding event has expired.

In one embodiment of the invention, the method for estimating workload will include a method step of determining if stationary or nearly stationary. The vehicle will be considered to be stationary if the vehicle is at standstill, that is has a zero speed, for a period of time exceeding a time period threshold value. The time period threshold value is preferably set to between 10 - 40 seconds. The vehicle will be considered to be stationary only first after the workload estimator has identified that the vehicle has been at standstill for a period exceeding the threshold value.
The vehicle will be considered to be nearly stationary if a vehicle speed input signal is below a predetermined vehicle speed threshold value for a time period exceeding a time period threshold value. The time period threshold value for the condition "nearly stationary" may be the same as the time period threshold value for the condition "stationary. However, a different longer time period threshold value may be used for the condition "nearly stationary" than for the condition "stationary". The time period threshold value for the condition "nearly stationary" is preferably set to between 20 s and 2 minutes for a vehicle speed threshold value of 4 kilometres per hour. If the workload estimator has determined that the vehicle is stationary or nearly stationary, the means for generating an output signal generates a low workload output signal regardless of if a demanding event has initiated a high workload output signal or not.

After evaluating if the vehicle is stationary or nearly stationary, the actual evaluation of the workload level will continue as set out above with reference to the flow chart of figure 5. In the event it is identified that the vehicle is stationary or nearly stationary it is possible to skip running through the remaining parts of the algorithm for determining the workload level since the workload estimator will generate a low workload output signal regardless of the status of the input signal indicative of tasks requiring driver attention. In this even no changes are performed until a new sampling of input signals is performed. It is also possible to run through the remaining part of the algorithm, without resetting the workload level and first thereafter perform a new sampling of input signals.

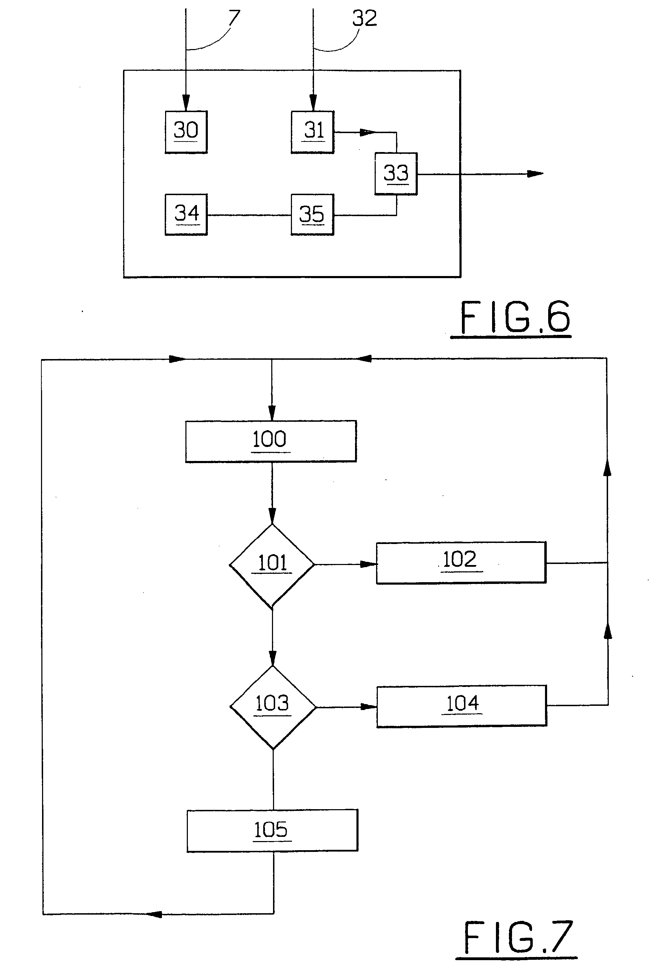

In figure 6 an embodiment of a workload estimator 2 according to the invention is shown. The workload estimator 2 includes means 30 for retrieving a signal 7 indicative of a task requiring driver attention, said signal being used to determine a workload level of an output signal of a workload estimator. The workload estimator may further include means 31 for identifying that the vehicle is stationary if the vehicle is at standstill for a period in time exceeding a threshold value. The condition that the vehicle is stationary may be identified from a speed signal 32 together with a timing condition, that parking or zero speed must has been ongoing for a period exceeding a threshold value. These means 31 may further identify if the vehicle is nearly stationary. The vehicle will be judged to be nearly stationary if the vehicle speed is less than a vehicle speed threshold value for a period of time exceeding a time period threshold value.

The workload estimator also includes means 33 for generating a workload output signal. The means for generating a workload output signal 33 will, in the event means for identifying if the vehicle is stationary or nearly stationary are included, generate a low workload output signal as long as vehicle remains stationary or nearly stationary regardless of if the signal indicative of a task requiring driver attention has initiated a high workload output signal or not.

The workload estimator further includes means for identifying a demanding event 34 from a signal indicative of a task requiring driver attention. The demanding event will occur between a demanding event starting point and a demanding event end point in time. The workload estimator also includes means 35 for generating a workload delay time frame. The workload delay time frame extends a period in time from said demanding event end point in time until a workload delay end point in time.
The means for generating a workload output signal 33 will generate a high workload output signal under the demanding event and maintain the high workload output signal under the workload delay time frame.

In figure 7 a flowchart of a method for determining workload according to an embodiment of the invention is shown. The method may be used in a workload manager 1 as shown in figure 1. According to this method a high workload output signal will be discriminated if the vehicle is stationary or nearly stationary. A low workload output signal will be generated regardless of if a demanding event is detected if the vehicle is stationary or nearly stationary. In a first method step 100 information regarding tasks requiring driver attention is retrieved from an input signal. This information also includes information relating to whether the vehicle is considered to be stationary or nearly stationary. In a second method 101 step it is determined if the vehicle is stationary or nearly stationary. The condition that the vehicle is stationary is identified if the vehicle is as standstill and has been at standstill for a period in time exceeding a threshold value. There are several ways of determining that a vehicle is at standstill. A straightforward possibility is to use the velocity signal and determine that a vehicle is at standstill if the signal indicates zero velocity. It is also possible to use a signal identifying the use of a parking brake. As soon as it is first identified if the vehicle is at standstill, for instance by noting that the vehicle has zero velocity, a timer starts running or alternatively a flag indicating a starting time is set. In the second method step 101 it is determined if the vehicle speed is zero, and in the event the vehicle speed is zero, for how long the vehicle speed has been zero. This is used by the timer, which started to run when first noting that the vehicle speed was zero or by comparing a value attached to the flag with a timer who set the flag.

If it is noted that the vehicle speed has been zero for a period exceeding a threshold value, preferably 10 - 40 seconds, the vehicle is considered to be stationary and a low workload signal is generated at a third method step 102 regardless of whether a demanding event has initiated a high workload output signal or not.

In the event the method includes detection of a condition identified as nearly stationary, the vehicle speed will be determined in the method step 101. In the event the vehicle speed has been identified to be below a vehicle speed threshold value for a time period exceeding a time period threshold value, the vehicle will be considered to be nearly stationary.

If the vehicle is considered to be nearly stationary a low workload signal is generated at a third method step 102 regardless of whether a demanding event has initiated a high workload output signal or not.

If the vehicle is neither stationary nor nearly stationary the method continues with estimating the workload of the current traffic situation. This is done in a fourth method step 103 by comparing signals indicative of tasks requiring driver attention with threshold values or by noting that a task requiring driver attention is ongoing.
If a demanding event is identified, that is if a signal relating to certain types of tasks exceeds a threshold value or if a signal relating to other tasks indicates that a task requiring driver attention is ongoing, a high workload output signal is generated at a fifth method step 104, otherwise a low workload output signal is generated at a sixth method step 105.

The method is restarted at the first method step 100 with a new set of collected data after having finished the third method step 102, the fifth method step 104 or the sixth method step 105.

The workload estimator and its means may be constituted by a state of the art microcomputer having a signal input gate, a signal output gate, processors capable of handling logic structure and timers or reference clock signals. The input gate can be arranged to handle analog as well as digital signals. The output gate can optionally be an analog or digital output gate. Preferably the input gate is coupled to a CAN bus. In this event, the input gate need only be able to handle digital signals. The workload estimator can also be constituted by a logic circuit including transistor elements arranged to perform the necessary signal comparisons and logic decisions.

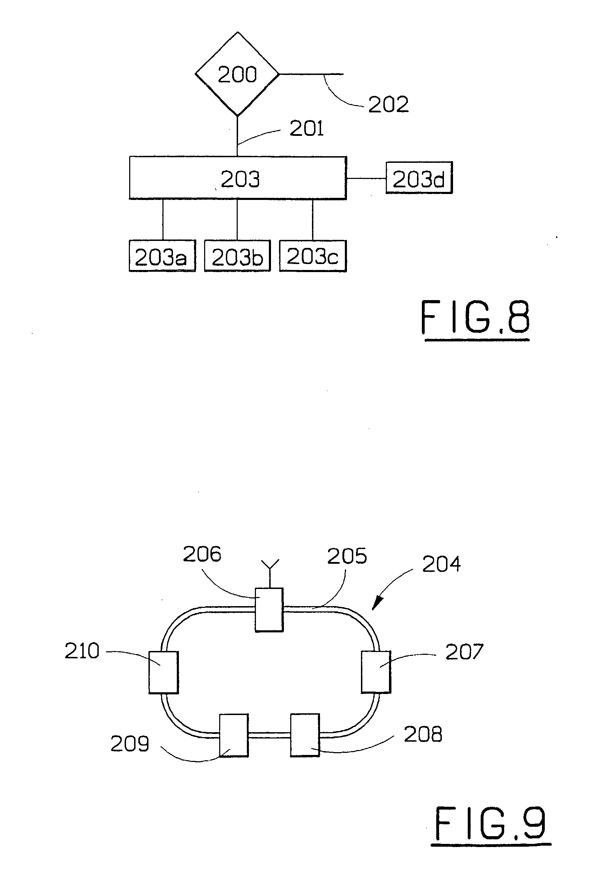

In figures 8 and 9 a method for handling incoming calls and a vehicle on board arrangement for handling an incoming call is described. According to this method and arrangement, an incoming call will be inhibited for a period of time if a high workload output signal is generated by a workload estimator at reception of the call.

Figure 8 shows a flow chart of a method for handling an incoming call to a telephone connected to an internal data communication system of a vehicle. At a first method step 200 the workload for the driver is estimated. The workload estimation can be performed by any known workload estimation technique. However, preferably a workload estimator as disclosed below will be used. The workload estimator will generate a high workload output signal 201 in the event a demanding event is detected in the workload estimation. In the event a demanding event is not detected, a low workload output signal 202 will be generated.

In the event a high workload signal is present at reception of the incoming call, the incoming call is in a second method step maintained connected for a period of time from reception of the incoming call while inhibiting access to said incoming call as long as a high workload signal is present under said period of time.

If the workload estimator change its output signal from indicating high workload to in stead indicate low workload before the predetermined period of time runs out, access to the telephone call will be allowed at a third step 203a.

If the high workload signal remains when said predetermined period of time runs out, according to different embodiments of the invention one of the following method steps will be taken:

According to a first embodiment the incoming call is disconnected in a third method step 203b if a high workload output signal is present when said period of time runs out.

According to a second embodiment the incoming call is routed to an answering machine in a third method step 203c if a high workload output signal is present when said period of time runs out. Optionally, the answering machine informs the caller that the driver is in a demanding event.

According to a third embodiment t access to the incoming call is allowed in a third method step 203d regardless of whether a high workload output signal is present or not when said period of time runs out

The predetermined period of time has preferably a duration of between 2 - 20 seconds. In a still more preferred embodiment the duration is set to between 4 - 6 seconds.
According to one embodiment of the invention, the access to the incoming call is inhibited by turning off the phone signal.

If a call has already been received before a demanding event occurs, the call will not be interrupted.

In figure 9 a vehicle on board arrangement 204 for handling an incoming call or is shown. The vehicle on board arrangement include an internal data communication system 205 of a vehicle. The internal data communication system can be arranges as a multiplex system as for instance a CAN bus. The internal data communication system is controlled by a central processor unit 210. The internal data communication system 205 is connected to a telephone 206 and a workload estimator 207.The workload estimator 206 may be of any type know to a person skilled in the art, but is preferably of the type which is disclosed in more detail below. The workload estimator 206 is arranged to generate a high workload output signal in the event the workload estimator 206 detects a demanding event. The arrangement 204 further includes means 208 for inhibiting access to said incoming call for a period of time in the event a high workload signal is present at the reception of the incoming call. The means for inhibiting access can be included in the workload estimator 207, in an information filter included in a workload manager as described below, or be separately arranged, for instance as means for inhibiting the ring signal arranged in connection with a loudspeaker arrangement which normally produces the ring signal at reception of a call.

The means for inhibiting access operates according to the method disclosed in relation to figure 1.
The mean for inhibiting access are arranged according to the following:

The vehicle on board arrangement includes means for maintaining the incoming call connected for a period of time from reception of the while, in the event a high workload signal is present at reception of the incoming call, inhibiting access to said incoming call as long as a high workload signal is present under said period of time. These means can be arranged by allowing the incoming call to be connected while letting said means for inhibiting the ring signal to cancel the ring signal.

The means for inhibiting access 208 is according to one embodiment of the invention is arranged to disconnect the incoming call if a high workload output signal is present when said period of time runs out.

The means for inhibiting access 208 is according to another embodiment of the invention is arranged to route the incoming call to an answering machine if a high workload output signal is present when said period of time runs out. Preferably, the arrangement 204 according to this embodiment includes or is connected to an answering machine 209, which has a communication path to the workload estimator. In the event the answering machine 209 is informed of that the workload estimator generates a high workload output signal, a message indication that the driver is under high workload will be generated.

The means for inhibiting access 208 is according to still another embodiment of the invention is arranged to allow access to the incoming call regardless of whether a high workload output signal is present or not when said period of time runs out

The predetermined period of time has a preferably a duration of between 2 - 10 seconds. In a still more preferred embodiment the duration is set between 4 - 6 seconds.

If a call has already been received before a demanding event occurs, the call will not be interrupted.

The invention is not restricted to the embodiments described above, but can be varied within the scope of the appended claims. In particular it is possible to amend the way the way data is collected and recollected. Furthermore it is possible to use the invention together with workload estimators which make use of physiological indicators. The invention is however preferably used together with workload estimators using performance related measures.

## Claims

1. Method for estimating workload for a driver of a vehicle comprising the following method steps:
- identifying a demanding event from a signal indicative of a task requiring driver attention, said demanding event occurring between a demanding event starting point and a demanding event end point in time, and
- generating a high workload output signal under said demanding event,
**characterised in that** the method includes the further steps of:
- generating a workload delay time frame which extends a period in time from said demanding event end point in time until a workload delay end point in time, and
- maintaining a high workload output signal under said workload delay time frame.

2. Method according to claim 1, **characterised in that** a demanding event is identified from said task requiring driver attention by comparing the signal value with a threshold value.

3. Method according to claim 2, **characterised in that** one or several of the following tasks are monitored for identifying demanding events: acceleration of the vehicle, steering angle and steering angle change rate.

4. Method according to claim 1, **characterised in that** a demanding event is identified from said task requiring driver attention by noting that an activity is ongoing.

5. Method according to claim 4, **characterised in that** one or several of the following tasks are monitored for identifying demanding events: brake pedal active, gear lever in rear position, turn indicator used and control buttons manoeuvred

6. Method according to any of the preceding claims, **characterised in that** at least two different tasks has different periods of duration of the workload delay time frame.

7. Method according to any of the preceding claims, **characterised in that** the method includes the further step of:
- inhibiting access to incoming calls and/or sms messages and/or internally generated messages for a period of time in the event a high workload signal is present at reception of the incoming call, sms message or internally generated message.

8. Workload estimator comprising:
- means for identifying a demanding event from a signal indicative of a task requiring driver attention, said demanding event occurring between a demanding event starting point and a demanding event end point in time, and
- means for generating a high workload output signal under said demanding event,
**characterised in that** the workload estimator further includes:
- means for generating a workload delay time frame which extends a period in time from said demanding event end point in time until a workload delay end point in time, and
- means for maintaining a high workload output signal under said workload delay time frame.

9. Workload estimator according to claim 8, **characterised in that** said means for identifying a demanding event includes a comparator which is arranged to compare the value of the signal indicative of a task requiring driver attention with a threshold value.

10. Workload estimator according to claim 9, **characterised in that** one or several of the following tasks are monitored for identifying demanding events: acceleration of the vehicle, steering angle and steering angle change rate.

11. Workload estimator according to claim 8, **characterised in that** said means for identifying a demanding event are arranged to identify a demanding event from said task requiring driver attention by noting that an activity is ongoing.

12. Workload estimator according to claim 11, **characterised in that** one or several of the following tasks are monitored for identifying demanding events: brake pedal active, gear lever in rear position, turn indicator used and control buttons maneuvered

13. Workload estimator according to any of claims 8-12. **characterised in that** at least two different tasks has different periods of duration of the workload delay time frame.

14. Workload estimator according to any of claims 8-13, **characterised in that** the workload estimator further includes means for inhibiting access to incoming calls and/or sms messages and/or internally generated messages for a period of time in the event a high workload signal is present at reception of the incoming call, sms message or internally generated message.

## Patentansprüche

1. Verfahren zum Schätzen einer Arbeitsbelastung eines Fahrzeugführers mit den folgenden Verfahrensschritten:
- Identifizieren eines fordernden Ereignisses aus einem Signal, das indikativ für eine Tätigkeit ist, die des Fahrers Aufmerksamkeit benötigt, wobei das fordernde Ereignis zwischen einem zeitlichen Startpunkt des fordernden Ereignisses und einem zeitlichen Endpunkt des fordernden Ereignisses auftritt, und
- Generieren eines Ausgangssignals für große Arbeitsbelastung unter dem fordernden Ereignis,
**dadurch gekennzeichnet, dass** das Verfahren die weiteren Schritte beinhaltet:
- Generieren eines Arbeitsbelastungs-Verzögerungszeitrahmens, welcher sich für eine Zeitspanne von dem zeitlichen Endpunkt des fordernden Ereignisses bis zu einem zeitlichen Endpunkt der Arbeitsbelastungs-Verzögerung erstreckt, und
- Aufrechterhalten eines Ausgangssignals für große Arbeitslast unter dem Arbeitsbelastungs-Verzögerungszeitrahmen.

2. Vorrichtung nach Anspruch 1,
**dadurch gekennzeichnet, dass** ein forderndes Ereignis aus der Tätigkeit identifiziert wird, die des Fahrers Aufmerksamkeit benötigt, indem der Signalwert mit einem Schwellwert verglichen wird.

3. Verfahren nach Anspruch 2,
**dadurch gekennzeichnet, dass** einer oder mehrere der nachfolgenden Tätigkeiten zum Identifizieren fordernder Ereignisse überwacht werden: Beschleunigung des Fahrzeuges, Steuerwinkel und Steuerwinkeländerungsrate.

4. Verfahren nach Anspruch 1,
**dadurch gekennzeichnet, dass** ein forderndes Ereignis aus einer Tätigkeit identifiziert wird, die des Fahrers Aufmerksamkeit benötigt, indem erfasst wird, dass eine Aktivität fortgeführt wird.

5. Verfahren nach Anspruch 4,
**dadurch gekennzeichnet, dass** eine oder mehrere der folgenden Tätigkeiten zum Identifizieren fordernder Ereignisse überwacht werden: Bremspedal tätig, Schalthebel in Rückfahrposition, Blinkeranzeige verwendet und Steuerschalter betätigt.

6. Verfahren nach einem der vorhergehenden Ansprüche,
**dadurch gekennzeichnet, dass** mindestens zwei verschiedene Tätigkeiten verschiedene Zeitspannen des Arbeitsbelastungs-Verzögerungszeitrahmens aufweisen.

7. Verfahren nach einem der vorhergehenden Ansprüche,
**dadurch gekennzeichnet, dass** das Verfahren den weiteren Schritt aufweist:
- Unterbinden eines Zugangs für eingehende Anrufe und/oder SMS-Botschaften und/oder intern generierte Botschaften für eine Zeitspanne in dem Fall, dass ein Signal für große Arbeitslast beim Empfang des eingehenden Anrufs, der SMS-Botschaft oder der intern generierten Botschaft vorliegt.

8. Arbeitslastschätzer mit:
- einer Einrichtung zum Identifizieren eines fordernden Ereignisses aus einem Signal, das indikativ für eine Tätigkeit ist, die des Fahrers Aufmerksamkeit benötigt, wobei das fordernde Ereignis zwischen einem zeitlichen Startpunkt des fordernden Ereignisses und einem zeitlichen Endpunkt des fordernden Ereignisses eintritt, und
- eine Einrichtung zum Generieren eines Ausgangssignals für große Arbeitslast unter dem fordernden Ereignis,
**dadurch gekennzeichnet, dass** der Arbeitslastschätzer ferner beinhaltet:
- eine Einrichtung zum Generieren eines Arbeitslast-Verzögerungszeitrahmens, der sich für eine Zeitspanne von dem zeitlichen Endpunkt des fordernden Ereignisses bis zu einem zeitlichen Endpunkt der Arbeitslastverzögerung erstreckt, und
- eine Einrichtung zum Aufrechterhalten eines Ausgangssignals für große Arbeitslast unter dem Arbeitslast-Verzögerungszeitrahmen.

9. Arbeitslastschätzer nach Anspruch 8,
**dadurch gekennzeichnet, dass** die Einrichtung zum Identifizieren eines fordernden Ereignisses einen Komparator beinhaltet, der zum Vergleich des Wertes des Signals, das indikativ für eine Tätigkeit ist, die des Fahrers Aufmerksamkeit benötigt, mit einem Schwellwert eingerichtet ist.

10. Arbeitslastschätzer nach Anspruch 9,
**dadurch gekennzeichnet, dass** eine oder mehrere der nachfolgenden Tätigkeiten zum Identifizieren fordernder Ereignisse überwacht werden: Beschleunigung des Fahrzeuges, Steuerwinkel und Steuerwinkeländerungsrate.

11. Arbeitslastschätzer nach Anspruch 8,
**dadurch gekennzeichnet, dass** die Einrichtung zum Identifizieren eines fordernden Ereignisses zum Identifizieren eines fordernden Ereignisses aus einer Tätigkeit, die des Fahrers Aufmerksamkeit benötigt, mittels einem Erfassen, dass eine Aktivität fortgeführt wird, eingerichtet ist.

12. Arbeitslastschätzer nach Anspruch 11,
**dadurch gekennzeichnet, dass** eine oder mehrere der folgenden Tätigkeiten zum Identifizieren fordernder Ereignisse überwacht werden: Bremspedal aktiv, Schalthebel in Rückfahrposition, Blinkeranzeige verwendet und Steuerschalter betätigt.

13. Arbeitslastschätzer nach einem der Ansprüche 8 bis 12,
**dadurch gekennzeichnet, dass** mindestens zwei verschiedene Tätigkeiten verschiedene Zeitspannen des Arbeitslast-Verzögerungszeitrahmens aufweisen.

14. Arbeitslastschätzer nach einem der Ansprüche 8 bis 13,
**dadurch gekennzeichnet, dass** der Arbeitslastschätzer ferner eine Einrichtung zum Verhindern eines Zugangs für eingehende Anrufe und/oder SMS-Botschaften und/oder intern generierte Botschaften für eine Zeitspanne, falls ein Signal für große Arbeitslast beim Empfang des eingehenden Anrufs, der SMS-Botschaft oder der intern generierten Botschaft vorliegt, aufweist.

## Revendications

1. Procédé pour estimer une charge de travail pour un conducteur d'un véhicule comportant les étapes de procédé suivantes consistant à :
- identifier un événement exigeant à partir d'un signal indicatif d'une tâche nécessitant l'attention du conducteur, ledit événement exigeant se produisant entre un point de début d'événement exigeant et un point de fm d'événement exigeant dans le temps, et
- produire un signal de sortie de charge de travail élevée sous ledit événement exigeant,
**caractérisé en ce que** le procédé inclut les étapes supplémentaires consistant à :
- produire un bloc de temps de retard d'une charge de travail qui prolonge une période de temps à partir dudit point de fin d'événement exigeant dans le temps jusqu'à un point de fin de retard d'une charge de travail dans le temps, et
- maintenir un signal de sortie de charge de travail élevée dans ledit bloc de temps de retard d'une charge de travail.

2. Procédé selon la revendication 1, **caractérisé en ce qu'**un événement exigeant est identifié à partir de ladite tâche nécessitant l'attention du conducteur en comparant la valeur de signal avec une valeur de seuil.

3. Procédé selon la revendication 2, **caractérisé en ce qu'**une ou plusieurs des tâches suivantes sont surveillées pour identifier des événements exigeants : accélération du véhicule, angle de braquage et vitesse de changement d'angle de braquage.

4. Procédé selon la revendication 1, **caractérisé en ce qu'**un événement exigeant est identifié à partir de ladite tâche nécessitant l'attention du conducteur en signalant qu'une activité est en cours.

5. Procédé selon la revendication 4, **caractérisé en ce qu'**une ou plusieurs des tâches suivantes sont surveillées pour identifier des événements exigeants : pédale de frein active, levier de vitesses en position de marche arrière, clignotant utilisé et boutons de commande manoeuvrés.

6. Procédé selon l'une quelconque des revendications précédentes, **caractérisé en ce qu'**au moins deux différentes tâches ont différentes périodes de durée du bloc de temps de retard d'une charge de travail.

7. Procédé selon l'une quelconque des revendications précédentes, **caractérisé en ce que** le procédé inclut l'étape supplémentaire consistant à :
- empêcher l'accès à des appels entrants et/ou des messages sms et/ou des messages générés en interne pendant une période de temps dans le cas où un signal de charge de travail élevée est présent à réception de l'appel entrant, du message sms ou du message généré en interne.

8. Dispositif d'estimation d'une charge de travail comportant :
- des moyens pour identifier un événement exigeant à partir d'un signal indicatif d'une tâche nécessitant l'attention du conducteur, ledit événement exigeant se produisant entre un point de début d'événement exigeant et un point de fin d'événement exigeant dans le temps, et
- des moyens pour produire un signal de sortie de charge de travail élevée sous ledit événement exigeant,
**caractérisé en ce que** le dispositif d'estimation d'une charge de travail inclut également :
- des moyens pour produire un bloc de temps de retard d'une charge de travail qui prolonge une période de temps à partir dudit point de fin d'événement exigeant dans le temps jusqu'à un point de fin de retard d'une charge de travail dans le temps, et
- des moyens pour maintenir un signal de sortie de charge de travail élevée dans ledit bloc de temps de retard d'une charge de travail.

9. Dispositif d'estimation d'une charge de travail selon la revendication 8, **caractérisé en ce que** lesdits moyens pour identifier un événement exigeant incluent un comparateur qui est agencé pour comparer la valeur du signal indicatif d'une tâche nécessitant l'attention du conducteur avec une valeur de seuil.

10. Dispositif d'estimation d'une charge de travail selon la revendication 9, **caractérisé en ce qu'**une ou plusieurs des tâches suivantes sont surveillées pour identifier des événements exigeants : accélération du véhicule, angle de braquage et vitesse de changement d'angle de braquage.

11. Dispositif d'estimation d'une charge de travail selon la revendication 8, **caractérisé en ce que** lesdits moyens pour identifier un événement exigeant sont agencés pour identifier un événement exigeant à partir de ladite tâche nécessitant l'attention du conducteur en signalant qu'une activité est en cours.

12. Dispositif d'estimation d'une charge de travail selon la revendication 11, **caractérisé en ce qu'**une ou plusieurs des tâches suivantes sont surveillées pour identifier des événements exigeants : pédale de frein active, levier de vitesses en position de marche arrière, clignotant utilisé et boutons de commande manoeuvrés.

13. Dispositif d'estimation d'une charge de travail selon l'une quelconque des revendications 8 à 12, **caractérisé en ce qu'**au moins deux différentes tâches ont différentes périodes de durée du bloc de temps de retard d'une charge de travail.

14. Dispositif d'estimation d'une charge de travail selon l'une quelconque des revendications 8 à 13, **caractérisé en ce que** le dispositif d'estimation d'une charge de travail inclut également des moyens pour empêcher l'accès à des appels entrants et/ou des messages sms et/ou des messages générés en interne pendant une période de temps dans le cas où un signal de charge de travail élevée est présent à réception de l'appel entrant, du message sms ou du message généré en interne.
